(19) Europäisches Patentamt
European Patent Office
Office européen des brevets

(11) Publication number: **0 194 352 A2**

(12) # EUROPEAN PATENT APPLICATION

(21) Application number: 85116063.0

(22) Date of filing: 11.01.86

(51) Int. Cl.[4]: G 01 N 33/531
C 07 K 15/00, C 07 D 493/10
//(C07D493/10, 311:00, 307:00)

A request for addition of five sheets of drawings has been filed pursuant to Rule 88 EPC. A decision on the request will be taken during the proceedings before the Examining Division.

(30) Priority: 19.12.84 US 683775

(43) Date of publication of application: 17.09.86 Bulletin 86/38

(84) Designated Contracting States: BE CH DE FR GB IT LI NL SE

(71) Applicant: ABBOTT LABORATORIES
14th Street and Sheridan Road North St
North Chicago Illinois 60064(US)

(72) Inventor: Ungemach, Frank Schneider
1221 S. Main St., Apt. 2B
Antioch, IL 60002(US)

(72) Inventor: Keegan, Candace Linda
671 Cardinal Court
Grayslake, IL 60030(US)

(72) Inventor: Nystrom, David Donald
4350 Finch Court, Apt. 8
Gurnee, IL 60031(US)

(72) Inventor: Stroupe, Stephen D.
606 Roosevelt Drive
Libertyville, IL 60048(US)

(74) Representative: Modiano, Guido et al,
MODIANO, JOSIF, PISANTY & STAUB Modiano & Associati Via Meravigli, 16
I-20123 Milan(IT)

(54) **Salicylate assay, tracers, immunogens and antibodies.**

(57) The present invention is directed to a fluorescent polarization assay for salicylate, to the various components needed for preparing and carrying out such an assay, and to methods of making these components. Specifically, tracers, immunogens and antibodies are disclosed, as well as methods for making them. The tracers and the immunogens are made from substituted benzoic acid compounds. A fluorescein moiety is included in the tracer, while a poly(amino acid) forms a part of the immunogen. The antibodies are prepared in response to the immunogen. The assay is conducted by measuring the degree of polarization retention of plane polarized light that has been passed through a sample containing antiserum and tracer.

EP 0 194 352 A2

# SALICYLATE ASSAY, TRACERS, IMMUNOGENS AND ANTIBODIES

## BACKGROUND OF THE INVENTION

### 1. Technical Field

The present invention relates to a method and reagents for a fluorescence polarization immunoassay procedure for determining the amount of salicylate in fluids, especially biological fluids such as serum, plasma or urine, and to a method of making the reagents. More specifically, the invention relates to (1) reagents (tracers and antibodies) for determining the amount of salicylate in a sample; (2) immunogen compounds used to raise antibodies; (3) synthetic methods (for making the tracer and immunogen compounds); and (4) analytical methods for conducting the assay.

### 2. Background Art

Salicylates are often prescribed as analgesics, antipyretics and anti-inflammatory agents and are generally considered to be relatively safe drugs. However, in cases of extreme dosages, salicylates can cause toxic side effects ranging from nausea, vomiting and tinnitis to fever and coma. The availability of over-the-counter preparations can lead to accidental or intentional overdoses. Moreover,

rheumatoid arthritis patients may need very high doses of salicylate because of severe pain associated with this type of chronic illness. Accordingly, in diagnosing cases of apparent overdose or in salicylate treatment of arthritic patients, it may be desirable to test or to monitor the levels of salicylate in urine, blood serum or plasma.

In the past, patient serum or plasma salicylate levels have typically been measured by colorimetric, spectrophotometric and spectrofluorometric assays. These methods are not without drawbacks. They usually require chemical extraction of drug from the serum/plasma matrix before analysis. Further, these methods are non-specific in that they can detect salicylate-like compounds as well as salicylates. High performance liquid chromatography (HPLC) and gas chromatography (GC) are used less frequently although they are very specific for detecting drug levels. HPLC and GC require extraction procedures and the assay time is lengthy.

In assays for other substances, competitive binding immunoassays have provided a more satisfactory alternative. Typically, competitive binding immunoassays are used for measuring ligands in a test sample. (For the purposes of this disclosure, a "ligand" is a substance of biological interest to be quantitatively determined by a competitive binding immunoassay technique.) The ligands compete with a labeled reagent, or "ligand analog," or "tracer," for a limited number of receptor binding sites on antibodies specific to the ligand and ligand analog. The concentration of ligand in the sample determines the amount of ligand analog which binds to the antibody: the amount of ligand analog that will bind is inversely proportional to the concentration of ligand in the sample, because the ligand and the ligand analog each bind to the antibody in proportion to their respective concentrations.

Fluorescence polarization provides a quantitative means for measuring the amount of tracer-antibody conjugate produced in a competitive binding immunoassay. Fluorescence polarization techniques are based on the principle that a fluorescent labeled compound, when excited by plane polarized light, will emit fluorescence having a degree of polarization inversely related to its rate of rotation. Accordingly, when a tracer-antibody conjugate having a fluorescent label is excited with plane polarized light, the emitted light remains highly polarized because the fluorophore is constrained from rotating between the time that light is absorbed and emitted. In contrast, when an unbound tracer is excited by plane polarized light, its rotation is much faster than the corresponding tracer-antibody conjugate and the molecules are more randomly oriented. As a result, the light emitted from the unbound tracer molecules is depolarized.

Such fluorescence polarization techniques have been applied in U.S. Patent No. 4,420,568 to Wang, et al., which is directed to the use of a triazinylamino-fluorescein moiety as the fluorophore. The present invention offers an advance in the art beyond the Wang, et al. patent, in that highly sensitive tracers, a method for making the tracers, and an assay using the tracers are provided specifically for the determination of salicylate. The assay conducted in accordance with the present invention is particularly accurate, as will be explained below.

## SUMMARY OF THE INVENTION

The present invention is directed to a fluorescence polarization assay for salicylate; to tracers, immunogens and antibodies for use in the assay; and to methods for making the tracers, immunogens and antibodies.

A first aspect of the invention relates to the discovery of unique tracers and immunogens having novel structures. According to the first aspect of the invention, the tracers and the immunogens can both be represented by the structural formula shown in Figure 5 where:

Q is a poly(amino acid), a poly(amino acid) derivative, fluorescein, or a fluorescein derivative;

X is NH or CO;

n is 0, 1 or 2;

R can be attached to any of the carbon atoms in the benzene ring other than those carbon atoms attached to Y or COW, and where R is a linking group including up to 2 heteroatoms when Q is a poly(amino acid) or a poly-(amino acid) derivative and up to 4 heteroatoms when Q is fluorescein or a fluorescein derivative, and having a total of from 0 to 8 carbon atoms and heteroatoms;

W is OH, or any OH salt;

Y is OH, $NH_2$, $CH_3$, F, Cl, Br or H when Q is fluorescein or a fluorescein derivative, and Y is OH, $NH_2$, $CH_3$, F or Cl when Q is a poly(amino acid) or a poly(amino acid) derivative; and

$Z_1$, $Z_2$ and $Z_3$ are each independently H or F when Q is fluorescein or a fluorescein derivative, and are each H when Q is a poly(amino acid) or a poly(amino acid) derivative.

When Q is a poly(amino acid) or a derivative thereof, the compound can be used as an immunogen. When Q is a fluorescein or a derivative thereof, the compound can be used as a tracer.

A second aspect of the invention relates to antibodies raised by the novel immunogen. According to

the second aspect of the invention, antibodies are prepared in response to a compound according to Claim 1 when Q is a poly(amino acid) or a derivative thereof.

According to a third aspect of the invention, an immunogen is made by a method comprising the step of coupling a compound represented by the structural formula shown in Figure 2, where:

R can be attached to any of the carbon atoms in the benzene ring other than those carbon atoms attached to Y or COW, and where R is a linking group including up to 2 heteroatoms and having a total of from 0 to 8 carbon atoms and heteroatoms;

W is OH, or any OH salt;

X is $NH_2$, COOH, CN, CHO, Br, I or OH; and

Y is OH, $NH_2$, $CH_3$, F or Cl;

with a poly(amino acid) or a derivative of a poly(amino acid).

According to a fourth aspect of the invention, a method is provided for making a tracer by coupling a compound represented by the structural formula shown in Figure 3, where:

R can be attached to any of the carbon atoms in the benzene ring other than those carbon atoms attached to Y or COW, and where R is a linking group including up to 4 heteroatoms and having a total of from 0 to 8 carbon atoms and heteroatoms;

W is OH, or any OH salt;

X is $NH_2$, COOH, CN or OH;

Y is OH, $NH_2$, $CH_3$, F, Cl, Br or H; and

$Z_1$, $Z_2$ and $Z_3$ are each independently H or F;

with fluorescein or a derivative of fluorescein.

According to a fifth aspect of the invention, a process for measuring concentration of salicylate is provided. A sample is contacted with salicylate antiserum, and a fluorescein containing salicylate derivative capable of producing a detectable fluorescence polarization response to the presence of the salicylate antiserum. Plane polarized light is then passed through the solution to obtain a fluorescence polarization response, and this response is detected as a measure of the amount of salicylate in the sample.

Further objects and attendant advantages of the invention will be best understood from a reading of the following detailed description taken together with the drawings and the Examples.

BRIEF DESCRIPTION OF THE DRAWINGS

In the following Figures the symbol "Fl" represents a fluorescein moiety, and the various other symbols are noted in the Detailed Description. It should be noted that in Figures 2, 3, 5, 6, 7 and 10, the various groups other than "Y" and "COW" can be located at any position of the benzene ring not occupied by the "Y" and "COW" substituents.

Figure 1 shows the general structure of the class of salicylates to be quantitatively determined in accordance with the present invention.

Figure 2 shows a class of reactants for a method of making an immunogen in accordance with the present invention.

Figure 3 shows a class of reactants for a method of making a tracer in accordance with the present invention.

Figure 4 shows the alternate structural formulae and names of the fluorescein moiety included in the tracers of the present invention.

Figure 5 shows a general structural formula for the tracers and the immunogens of the present invention.

Figure 6 shows a general structural formula for the immunogens of the present invention.

Figure 7 shows a general structural formula for the tracers of the present invention.

Figure 8 shows a structural formula for preferred immunogens of the present invention.

Figure 9 shows a structural formula for preferred tracers of the present invention.

Figure 10 shows a precursor for the immunogens shown in Figures 6 and 8 and for the tracers shown in Figures 7 and 9.

Figure 11 shows various linkages that couple the fluorescein moiety to the precursor at the X position in Figure 10, when Figure 10 represents a precursor for the tracers shown in Figures 7 and 9.

Figures 12 through 16 show various examples of structures and names of tracers in accordance with the present invention.

Figures 17 through 20 show various examples of structures and names of hapten reactants used to form the immunogens of the present invention.

## DETAILED DESCRIPTION OF THE PREFERRED EMBODIMENTS

The various aspects of the invention will now be discussed in relation to the Figures and/or the Examples.

The present invention involves the use of fluorescein and derivatives of fluorescein. In particular, a necessary property of fluorescein and its derivatives for the usefulness of the tracer compounds is the fluorescence of fluorescein. Fluorescein exists in two tautomeric forms, illustrated in Figure 4, depending on the acid concentration (pH) of the environment. In the open (acid) form, there are a number of conjugated double bonds which make that form of fluorescein (and compounds containing a fluorescein moiety) capable of absorbing blue light and emitting green fluorescence after an excited state lifetime of about 4

nanoseconds. When the open and closed forms coexist, the relative concentration of molecules in the open and closed forms is easily altered by adjustment of the pH level. Generally, the tracer compounds of the present invention exist in solution as biologically acceptable salts such as sodium, potassium, ammonium and the like, which allows the compounds to exist in the open, fluorescent form, when employed in the analytical methods of the present invention. The specific salt present will depend on the buffer employed to adjust the pH level. For example, in the presence of a sodium phosphate buffer, the compounds of the present invention will generally exist in the open form, as a sodium salt.

As used herein, the term "fluorescein," either as an individual compound or as a component of a larger compound, is meant to include both the open and closed forms, if they exist for a particular molecule, except in the context of fluorescence. An open form is necessary for the fluorescence to occur.

The numbering of carbon atoms of the fluorescein molecule varies, depending upon whether the open or closed form of the molecule is considered. Accordingly, the literature concerning fluorescein and its compounds is not uniform as to carbon atom numbering. In the closed form, the para-carbon to the carbonyl of the lactone on the phenyl ring is numbered 6. In the open form, the para-carbon to the carboxylic acid group on the phenyl ring is numbered 5. See Figure 4. For the purpose of this disclosure the numbering of the closed form is adopted because the raw materials used in the syntheses are most popularly numbered with that system. The carbon atom of fluorescein and its compounds which is opposite the carboxyl group is therefore numbered "6" for the purposes of the present disclosure.

A tracer which is not complexed to an antibody is free to rotate in less than the time required for absorption and re-emission of fluorescent light. As a result, the re-emitted light is relatively randomly oriented so that the fluorescence polarization of a tracer not complexed to an antibody is low, approaching zero. Upon complexing with a specific antibody, the tracer-antibody complex thus formed assumes the rotation of the antibody molecule which is slower than that of the relatively small tracer molecule, thereby increasing the polarization observed. Therefore, when a ligand competes with the tracer for antibody sites, the observed polarization of fluorescence of the tracer-antibody complex becomes a value somewhere between that of the tracer and tracer-antibody complex. If a sample contains a high concentration of the ligand, the observed polarization value is closer to that of the free tracer i.e., low. If the test sample contains a low concentration of the ligand, the polarization value is closer to that of the bound tracer i.e., high. By sequentially exciting the reaction mixture of an immunoassay with vertically and then horizontally polarized light and analyzing only the vertical component of the emitted light, the polarization of fluorescence in the reaction mixture may be accurately determined. The precise relationship between polarization and concentration of the ligand to be determined is established by measuring the polarization values of calibrators with known concentrations. The concentration of the ligand can be extrapolated from a standard curve prepared in this manner.

The particular tracers formed in accordance with this invention have been found to produce surprisingly good assays, as will be demonstrated later in this disclosure.

1. The Reagents

Both the immunogens and the tracers of the present invention can be represented by the general structural formula set forth in the Summary of the Invention, and illustrated in Figure 5. When Q is a poly(amino acid), the structure represents the immunogen; when Q is a fluorescein moiety, the structure represents the tracer.

The objective is to have competition between salicylate and the tracer for the recognition sites of the antibody. Great variations in the structure of the haptens and tracers are allowed in achieving this goal. For the purposes of this invention, "haptens" are precursors of the immunogens, comprising generally a substituted benzoic acid derivative and a linking group to the poly(amino acid) carrier.

a. The Structure of the Immunogens

Usable antibodies can be produced from a variety of benzoic acid derivatives. Immunogens made from compounds that have OH, $NH_2$, $CH_3$, F or Cl in place of the -OH on salicylic acid can produce antibodies in animals; such antibodies are useful in a salicylate assay according to the invention when combined with the appropriate tracer.

The immunogens of the present invention have the general structural formula shown in Figure 6, and in the preferred form of the invention, the immunogens have the structural formula shown in Figure 8. The immunogens can be prepared by coupling a compound of the class shown in Figure 2 with a poly(amino acid) or a derivative of a poly(amino acid), as will be discussed in the context of the synthetic method and the Examples below.

In a preferred form of the invention, the immunogen has the structural formula shown in Figure 8.

This structure is preferred because the best recognition of the OH and COW groups occurs when the ring is substituted at a position as distant as possible from these groups. Although substitution at either the 4 or 5 positions is equivalently preferred from a structural point of view, starting materials are more readily available and the synthesis itself is greatly simplified for the 5 position on the ring. Although bovine serum albumin is the poly(amino acid) in this preferred form, it should be understood that various protein carriers may be employed, including albumins, serum proteins, e.g., globulins, ocular lens proteins, lipoproteins and the like. Illustrative protein carriers include bovine serum albumin, keyhole limpet hemocyanin, egg ovalbumin, bovine gamma-globulin, thyroxine binding globulin, etc. Alternatively, synthetic poly(amino acids) may be prepared having a sufficient number of available amino groups such as lysines.

b. The Structure of the Tracers

The possible variations in the structure of the tracers of the invention are even greater than the possible variations in the structure of the haptens thereof. The tracers of the present invention have the general structural formula shown in Figure 7, where Fl represents a fluorescein moiety or a fluorescein derivative. In a preferred form of the invention, the tracers have the structural formula shown in Figure 9.

The tracer is a benzoic acid derivative that is linked to a fluorescein derivative by, for example, an amido, amidino, triazinylamino, carbamido, thiocarbamido, carbamoyl, thiocarbamoyl, or sulfonylcarbamoyl group, as shown in Figure 11. The tracers are prepared by linking the appropriate fluorescein derivative to a benzoic acid derivative containing an amino, carboxylic

acid, hydroxy, imidate, hydrazide, isocyanate, thioisocyanate, chloroformate, chlorothioformate, chlorosulfonylcarbamoyl, or the like group, as will be discussed in the context of the synthetic method and the Examples below.

By way of example, any of the following fluorescein derivatives can be used:

$Fl-NH_2$ fluorescein amine

$Fl-CO_2H$ carboxyfluorescein

$Fl-NHCOCH_2I$ α-iodoacetamidofluorescein

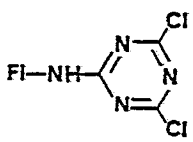

(DTAF) 2,4-dichloro-1,3,5,-triazin-2-yl amino-fluorescein

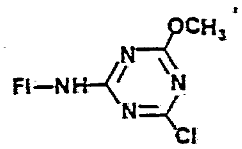

4-chloro-6-methoxy-1,3,5-triazin-2-ylamino fluorescein

Fl-NCS fluorescein thioisocyanate

2. The Antibodies

The antibodies of the present invention are prepared by developing a response in animals to the immunogens described above. The immunogen is administered to animals such as rabbits or sheep by a series of injections, in a manner well-known to those skilled in the art.

3. Synthetic Methods

Both the immunogens and the tracers of the present invention can be made from a precursor having the general structural formula shown in Figure 10, where

Y is OH, $NH_2$, $CH_3$, F, Cl, Br or H when the preparation is directed to a tracer, and Y is OH, $NH_2$, $CH_3$ F or Cl when the preparation is directed to an immunogen;

W is OH or any salt of OH;

X is $NH_2$, COOH, CHO, CN, Br, I or OH when the preparation is directed to an immunogen, and $NH_2$, COOH, CN or OH when the preparation is directed to a tracer; and

$Z_1$, $Z_2$ and $Z_3$ are each independently H or F when the preparation is directed to a tracer, and are each H when the preparation is directed to an immunogen.

a. The Synthesis of the Immunogens

The immunogens of the present invention are made by coupling a hapten, such as that shown by the general structure of Figure 2 when X is $NH_2$, COOH, CN, CHO, bromine, iodine or OH, to a poly(amino acid). The poly(amino acid) moiety can be linked to the hapten by an amide, an amidine, an alkyl, a urea, a thiourea, a carbamate, or a thiocarbamate linkage. In a preferred embodiment, the poly(aminoacid) is bovine serum albumin (BSA), and the hapten is shown in Figure 17. The hapten is preferably coupled under conditions normally used to form an amide linkage, and such conditions are well known to those skilled in the art. It is most preferred that active ester procedures be used, as these are the most effective for forming the desired amide linkage in this context.

The immunogens are prepared by coupling a hapten that contains an $-NH_2$, $-CO_2H$, $-CONHNH_2$, -CNOR, -CHO, -Br, -I, -NCO, -NCS, -OCOCl or -OCSCl group to a

poly(amino acid). The $-NH_2$ case can be coupled by forming a diazonium salt and adding this to the poly(amino acid) or by activating the carboxylic acid group on the polyamino acid in the presence of the $-NH_2$ group. The diazonium salt method only works for aromatic amines and is typically prepared by mixing the amine with sodium nitrite. In the case where $Y=NH_2$, protection followed by coupling and then deprotection of the $Y=NH_2$ would be necessary.

The activation of the carboxylic acid groups on the poly(amino acid) can be accomplished by mixing the hapten and the poly(amino acid) with 1-ethyl-3-(3-dimethylamino-propyl) carbodiimide (EDC), N,N'-dicyclohexylcarbodiimide (DCC), 1-cyclohexyl-3-(2-morpholinoethyl) carbodiimide metho-p-toluenesulfonate, or the like. The $-CO_2H$ case is also coupled by the activation method (EDC) or the active ester method, as described below in the tracer synthesis section. The $-CONHNH_2$ case is coupled in the same manner as for the non aromatic amino case. The -CNOR case is coupled directly to the poly(amino acid). The -CHO case is coupled to the poly(amino acid) by reductive amination. The poly(amino acid) is mixed with the -CHO hapten and the resulting imine is reduced with sodium cyanoborohydride to yield alkylated amines on the poly(amino acid). The -Br and -I cases also produce alkylated amines on the poly(amino acid), but by direct coupling of the alkylhalide to the amine on the poly(amino acid). The isocyanate (-NCO), isothiocyanate (-NSC), chloroformate (-OCOCl) and chlorothioformate (-OCSCl) cases produce urea, thiourea, carbamate and thiocarbamate linkages, respectively. This is accomplished by direct coupling of the hapten to the poly(amino acid).

The synthesis of the above haptens (immunogen precursors where Y=OH, $NH_2$, $CH_3$, F or Cl) are accomplished in very similar ways. Figure 2 shows an immunogen precursor class in accordance with a preferred embodiment of the method of the present invention. The

ideal starting material in general is the formyl derivative of the appropriate substituted benzoic acid; for example, 5-formyl-2-methylbenzoic acid. These aldehydes can be condensed with a variety of reagents that will allow different derivatives to be prepared. Reaction with the appropriate Wittig reagent [for example, $Br^{+} Ph_3P^{-}(CH_2)_nR$] followed by reduction can prepare homologous carboxylic acid ($-CO_2H$), nitrile (-CN) and aldehyde (-CHO) derivatives. The aldehyde and carboxylic acid groups would be initially protected as an acetal and ester, respectively, which are removed by hydrolysis.

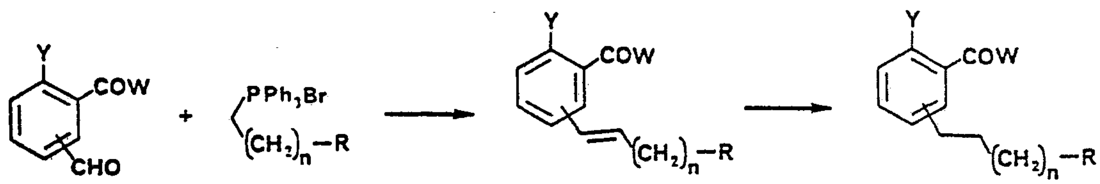

Wittig reagents containing heteroatoms, unsaturations and/or branching can be used to prepare other derivative forms as desired. In the case where $Y=NH_2$, the starting aldehyde must have the amine protected. The best protection is for Y to be a nitro group. The nitro group would be reduced in the second step when the olefin is reduced to yield the amino group. This route is preferred over others due to the great synthetic flexibility it provides.

In the preparation of shorter carbon chain carboxylic acids or nitriles, the Knoevenagel reaction method is preferred. The aldehyde and malonic acid are condensed in the presence of a base, such as piperidine, morpholine, or the like, which results in the formation of an α,β- unsaturated carboxylic acid. This carboxylic

0194352

acid can be coupled or reduced to the corresponding saturated carboxylic acid by the methods described earlie and then coupled. The nitrile is prepared by the same method with cyanoacetic acid instead of malonic acid. The nitrile is converted to the imidate, see below, and then coupled.

The carboxylic acid derivatives are ready to be coupled to produce immunogens. The nitrile derivatives are converted to alkoxy imidates by treating the nitrile with anhydrous alcohol and hydrogen chloride gas. The alkoxy imidates are then ready for coupling. The hydrazide derivatives are prepared from the corresponding carboxylic acid derivatives by active ester coupling with hydrazine or by reacting hydrazine with the corresponding carboxylic ester derivative. The aldehyde derivatives are also ready to be coupled to produce immunogens. They are also convertible to the corresponding amines. This is accomplished by reductive amination or by the preferred conversion to the corresponding oxime followed by reduction. For convenience, the unsaturated aldehydes obtained from the reaction of the Wittig reagent with the original aldehydes are converted to the oxime; then the reductions of the olefin, oxime and, for $Y=NO_2$, the nitro group, are effected simultaneously. These amino derivatives are then ready to be coupled to produce immunogens. With the appropriate protection of the -Y and -COW groups, the amine is convertible to an isocyanate or thioisocyanate group by reaction of the amine with phosgene or thiophosgene. The isocyanate and thioisocyanate derivatives are then coupled to a poly(amino acid) and the protecting groups are removed.

The aldehyde derivatives can also be convertec to the corresponding alcohols by reduction. This can be accomplished with sodium borohydride, hydrogenation,

or the like. The alcohols can be converted to the bromo or iodo derivatives with phosphorous tribromide, phosphorous triiodide, or the like; also, the iodo derivative can be prepared from the bromo derivative by halogen exchange with sodium iodide or the like. The halo derivatives are then ready to be coupled. The nitrile derivatives can also be prepared from the halo derivatives by reaction with cyanide.

The aldehydes can be condensed with (aminohydroxy)alkylcarboxylic acids, such as $NH_2OCH_2CO_2H$, to produce substituted oxime derivatives. These are ready to be coupled to a poly(amino acid). The oxime alkyl carboxylic acid derivatives can be partially reduced to the corresponding (aminohydroxy)alkylcarboxylic acid derivatives, which also are ready to be coupled to a poly(amino acid). The same type of condensation and reduction can be accomplished with hydrazine and hydrazine derivatives.

The alcohol derivatives can also be converted to the chloroformate and chlorothioformate derivatives. This is accomplished by reacting the alcohol, with Y = OH or $NH_2$ and COW protected, with phosgene or thiophosgene. The resulting chloroformates or chlorothioformates are coupled to a poly(amino acid) and then the protecting groups are removed.

The case of Y=OH provides a unique situation that allows easy access to carboxylic acid derivatives. The Friedel-Crafts reaction of a cyclic anhydride with salicylic acid followed by reduction of the resulting ketone group with sodium borohydride results in alkyl carboxylic acid derivatives. This only works for 5-substituted alkyl carboxylic acid derivatives of salicylic acid.

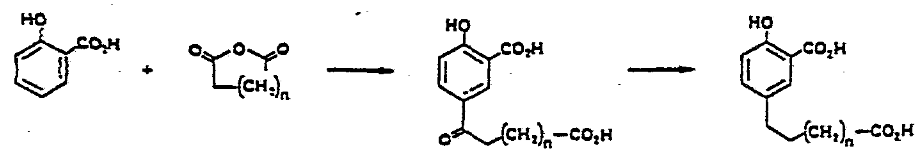

The formyl derivatives are easily prepared from the halomethyl derivatives. This is accomplished by oxidation, for example, with silver nitrate or 2-nitropropane, or the like, or by conversion to the corresponding hydroxymethyl derivative followed by oxidation to the aldehyde, for example, with pyridinium chlorochromate, pyridinium dichromate, or the like. The halomethyl derivatives can be prepared by halogenation of the corresponding methyl derivative by the standard N-bromosuccinimide method, or the like. The methyl derivatives are all known compounds and many are commercially available.

In the case where $Y=CH_3$, a mixture of halogenated products are obtained. The desired bromomethyl derivative is not easily purified, so the mixture is converted to the corresponding hydroxymethyl derivative which can be easily purified (see M. Nakazaki et al J. Org. Chem 1980, 45, 1428-35). The alcohol can then be oxidized to the desired aldehyde as mentioned earlier.

b. The Synthesis of the Tracers

The tracers of the present invention are made by coupling a fluorescein moiety, or a derivative of fluorescein, to the general structure shown in Figure 10 when X is $NH_2$, COOH, CNOR or OH. The fluorescein moiety can be linked to the amino, carboxyl, imidate or alkoxy functional group by an amide, an amidine, a urea, a thiourea, a carbamate, a thiocarbamate, triazinylamino or sulfonylcarbamate linkage, as shown in Figure 11. In the presently preferred embodiment, the fluorescein

derivative is 6-carboxyfluorescein (available from Calbiochem, La Jolla, Calif.), and this is coupled to a precursor shown in Figure 3. The 6-carboxyfluorescein is coupled to 5-(aminomethyl)salicylic acid by first forming the active ester of 6-carboxyfluorescein. The preferred active ester is N-hydroxysuccinimide active ester and the preferred method is via N,N'-dicyclohexylcarbodiimide activation in dry pyridine. Other activating groups, such as 1-hydroxybenzotriazole, p-nitrophenol, and carbonyldiimidazole, can be used; and other solvents, such as dimethylformamide and dimethylsulfoxide, can be used. The reactants are preferably coupled under conditions for forming amide linkages, and it is most preferred that active ester procedures be used. Usable tracers can be prepared from a variety of benzoic acid derivatives.

All benzoic acid derivatives that have a terminal amino group, such as amino, hydrazinyl, hydrazido or the like, are coupled to carboxyfluorescein by the active ester method or the mixed anhydride method, and coupled to fluorescein isothiocyanate, DTAF or alkoxy DTAF by simply mixing the two materials in solution. The amino group can be converted to the isocyanate and thioisocyanate groups by reaction with phosgene and thiophosgene, respectively. These are then condensed with aminofluorescein to produce the tracer. The Y=OH,$NH_2$ and COW groups require a protecting group for the conversion of the amino group to the isocyanate and the thioisocyanate groups which is removed after the coupling to fluorescein amine is complete.

All benzoic acid derivatives that have a terminal carboxylic acid group, such as carboxylic acid, (aminohydroxy)alkylcarboxylic acid or the like, are coupled to aminofluorescein by the active ester method.

All benzoic acid derivatives that have a terminal hydroxy group can be coupled to fluorescein by

reaction with DTAF, α-iodoacetamidofluorescein or fluorescein isothiocyanate in solution. The hydroxy group can be converted to the chlorosulfonylcarbamoyl, chloroformate and chlorothioformate groups by reaction with chlorosulfonylisocyanate, phosgene and thiophosgene, respectively. These derivatives are then coupled to aminofluorescein in solution to produce the tracer. The Y=OH,$NH_2$ and COW groups require a protecting group for the conversion of the hydroxy group to the chlorosulfonylcarbamoyl, chloroformate and chlorothioformate groups which is removed after the coupling to fluorescein amine is complete.

All benzoic acid derivatives that have a terminal nitrile group are converted to imidates in anhydrous alcohol in the presence of hydrogen chloride gas. The imidate is then coupled to fluorescein amine in solution to prepare the tracer.

The preparation of the various amino, carboxylic acid, hydroxy and nitrile derivatives of the benzoic acid derivatives, with the exception of Y=Br,H and $Z_1$, $Z_2$ and/or $Z_3$=F, were described above in the immunogen preparation section. The derivatives where Y=Br or H are prepared by the same method as Y=Cl. The derivatives where $Z_1$, $Z_2$ and/or $Z_3$=F are prepared from the corresponding amino derivatives. The amino derivatives are prepared by reduction of the corresponding nitro derivatives. The nitro derivatives are prepared from known benzene derivatives by known methods.

## 4. The Assay

The particular tracers and antibodies of the present invention have been found to produce surprisingly good results in fluorescence polarization assays for salicylates. Figure 1 shows the general structure of the class of salicylates that can be quantitatively

determined in accordance with the present invention. The assay of the present invention provides a more rapid salicylate assay method than prior art methods, because it requires no specimen treatment before analysis and because the assay system has minimal cross-reactivity to salicylate-like compounds.

In accordance with the analytical methods of the present invention, i.e. the methods of determining salicylate by a fluorescence immunoassay procedure using the tracer compounds and immunogens of the invention, a sample containing or suspected of containing salicylate is intermixed with a biologically acceptable salt of a tracer and an antibody specific to salicylic acid and the tracer. The antibody is produced using the immunogen as described above. The salicylate and tracer compete for limited antibody sites, resulting in the formation of complexes. By maintaining constant the concentration of tracer and antibody, the ratio of salicylate-antibody complex to tracer-antibody complex that is formed is directly proportional to the amount of salicylate in the sample. Therefore, upon exciting the mixture with linearly polarized light and measuring the polarization of the fluorescence emitted by a tracer and a tracer-antibody complex, one is able to quantitatively determine the amount of salicylate in the sample.

The results can be quantified in terms of net millipolarization units, span (in millipolarization units) and relative intensity. The measurement of millipolarization units indicates the maximum polarization when a maximum amount of the tracer is bound to the antibody in the absence of any salicylate. The higher the net millipolarization units, the better the binding of the tracer to the antibody.

The span is an indication of the difference between the net millipolarization and the minimum amount of tracer bound to the antibody. A larger span provides for a better numerical analysis of data.

The intensity is a measure of the strength of the signal above background. Thus, a higher intensity will give a more accurate measurement. The intensity is determined at about 0.9 to 1.1 nanomolar for the preferred tracers of the invention, as the sum of the vertically polarized intensity plus twice the horizontally polarized intensity. The intensity can range from a signal from about three times to about thirty times the background noise, depending upon the concentration of the tracer and other assay variables. For the purposes of the present invention, an intensity aimed roughly at about eight to ten times that of noise background is preferred.

Table I shows the results obtained with various embodiments of the present invention, in terms of span, millipolarization units and intensity. In all instances, bovine serum albumin (BSA) was used as the protein carrier. As seen from the data in Table I, an assay produced from an immunogen made from the hapten of Figure 17 used in combination with the tracer of Figure 12 provides excellent results. Accordingly, this combination is presently the most preferred form of the invention. In addition, the hapten/tracer combinations represented by the combinations of Figures 17 and 16, Figures 17 and 14, Figures 17 and 13 and Figures 20 and 13 also produced acceptable results and are alternative preferred combinations.

TABLE I

| Hapten used in Immunogen for Antibody | Tracer | Net Polarization* | Span* | Intensity** |
|---|---|---|---|---|
| FIG. 20 | FIG. 16 | 226 | 55 | 13 |
| FIG. 20 | FIG. 15 | 196 | 6 | 10 |
| FIG. 20 | FIG. 14 | 210 | 81 | 21 |
| FIG. 20 | FIG. 13 | 224 | 100 | 16 |
| FIG. 20 | FIG. 12 | 216 | 72 | 14 |
| Diazonium Salt of FIG. 19 | FIG. 16 | 234 | 18 | 7 |
| FIG. 18 | FIG. 16 | - | *** | - |
| FIG. 18 | FIG. 15 | - | *** | - |
| FIG. 18 | FIG. 14 | 272 | 20 | 20 |
| FIG. 18 | FIG. 13 | 203 | 37 | 14 |
| FIG. 18 | FIG. 12 | 305 | 57 | 8 |
| FIG. 17 | FIG. 16 | 210 | 104 | 13 |
| FIG. 17 | FIG. 15 | 204 | 69 | 12 |
| FIG. 17 | FIG. 14 | 228 | 102 | 19 |
| FIG. 17 | FIG. 13 | 219 | 110 | 9.5 |
| FIG. 17 | FIG. 12 | 276 | 128 | 11 |

* In millipolarization units

** This is expressed as a ratio of net intensity to background noise

*** Binding of antibody to tracer too low to use for span testing

The pH at which the method of the present invention is practiced must be sufficient to allow the fluorescein moiety of the tracers to exist in their

open form. The pH may range from about 3 to 12, more usually in the range of from about 5 to 10, most preferably from about 6 to 9. Various buffers may be used to achieve and maintain the pH during the assay procedure. Representative buffers include borate, phosphate, carbonate, tris, barbital and the like. The particular buffer employed is not critical to the present invention, but the tris and phosphate buffers are preferred. The cation portion of the buffer will generally determine the cation portion of the tracer salt in solution.

The preferred method of the improved assay of the present invention will now be discussed in detail. The assay is a "homogeneous assay," which means that the end polarization readings are taken from a solution in which bound tracer is not separated from unbound tracer. This is a distinct advantage over heterogeneous immunoassay procedures such as those where the bound tracer must be separated from the unbound tracer before a reading can be taken.

The reagents for the fluorescence polarization assay of the present invention comprise antibody, specific for salicylate, tracer and salicylate pre treatment. Additionally, largely conventional solutions including a dilution buffer, salicylate calibrators and salicylate controls are desirably prepared. Typical solutions of these reagents, some of which are described below, are commercially available in assay "kits" from Abbott Laboratories, Abbott Park, Illinois.

All percentages expressed herein are weight/-volume unless otherwise indicated. The tracer formulation presently preferred is 78 nanomolar tracer in: 0.1 molar tris buffer at pH 7.5; 0.1% sodium dodecyl sulfate; 0.1% sodium azide; and 0.01% bovine gamma-globulin. The antiserum formulation comprises rabbit

serum diluted with: 0.1 molar tris buffer at pH 7.5; 0.1% sodium azide; 0.01% bovine gamma-globulin; and 2% ethylene glycol (volume/volume). The dilution buffer comprises: 0.1 molar sodium phosphate at pH 7.5; 0.1% sodium azide; and 0.01% bovine gamma-globulin. The pretreatment formulation comprises:

0.1 molar tris buffer at pH 7.5; 0.1% sodium azide; 0.1% sodium dodecyl sulfate; and 0.01% bovine gamma-globulin. Salicylate calibrators comprising salicylate in normal human serum at concentrations of 0.0, 50.0, 100.0, 200.0, 400.0 and 800.0 milligrams per liter, with 0.1% sodium azide as a preservative are useful. Salicylate controls comprising salicylate in normal human serum are provided at concentrations of 75.0, 300.0 and 600.0 milligrams per liter with 0.1% sodium azide as a preservative are also useful.

The preferred procedure is especially designed to be used in conjunction with the Abbott TDx®. Analyzer available from Abbott Laboratories, Irving, Texas. Fifty microliters of serum or plasma are required. The calibrators, controls, or unknown samples are pipetted directly into the sample well of the TDx® sample cartridge. One of the advantages of this procedure is that the sample does not require any special preparation.

The assay procedure from this point is fully automated.

If a manual assay is being performed, then the sample is mixed with the pretreatment solution in dilution buffer and a background reading is taken. The tracer is then mixed with the assay. The antibody is then finally mixed into the test solution. After incubation, a fluorescence polarization reading is taken.

The fluorescence polarization value of each calibrator, control or sample is determined and is printed on the output tape of an instrument such as the Abbott TDx® Analyzer. A standard curve is generated in the instrument by plotting the polarization of each calibrator versus its concentration using a nonlinear regression analysis. The concentration of each control or sample is read off the stored calibration curve and printed on the output tape.

With respect to the foregoing preferred procedure, it should be noted that the tracer, antibody, pretreatment solution, calibrators and controls should be stored between about 2 and about 8°C while the dilution buffer should be stored at ambient temperature. A standard curve and controls should be run every two weeks, with each calibrator and control run in duplicate. Controls should be run with each batch, and all samples can be run in duplicate.

It should be understood that the foregoing detailed description and the following Examples are intended to be illustrative, but not limiting, with respect to the scope of the present invention. Various modifications will become apparent to one skilled in the art, and thus it is intended that the scope of the invention be defined solely by the claims and legal equivalent thereof.

## EXAMPLES

Examples I through XI describe experiments that were performed in accordance with the concepts of the present invention. Example I is directed to preparation of an immunogen useful for producing antibody; Examples II through VII are directed to the synthesis of precursors for immunogens and tracers; and Examples VIII through XI are directed to the preparation of tracers.

Example I: Preparation of an Immunogen

Preparation of the activated ester for the preferred assay was made by dissolving in pyridine 107.6 milligrams of the compound illustrated in Figure 17; 158.4 milligrams of dicyclohexylcarbodiimide (DDC); and 87.7 milligrams of N-hydroxysuccinimide. The solution was stirred for approximately two hours at ambient temperatures. Dicyclohexylurea (DCU) forms as a byproduct of the reaction. After incubation, distilled water was added to the solution, forcing more DCU to precipitate. The solution was filtered before the addition of 5 milliliters of a solution of 117.8 milligrams of BSA in distilled water. The solution was stirred overnight at room temperature, and dialyzed against distilled $H_2O$ for two weeks before being purified further on a G-25 Sephadex column with 0.01m sodium phosphate buffer as the eluant.

Example II: 3-(3-carboxy-4-hydroxyphenyl)propenoic acid

1 gram of 5-formylsalicylic acid (Aldrich Chemical Co.) and 1.27 grams of malonic acid (Aldrich Chemical Co.) were dissolved in 13 ml pyridine (dried over KOH) and 1.1 ml morpholine. The mixture was heated at about 100°C for approximately 29 hours. Then the mixture was allowed to cool to room temperature, and the solvent was removed in vacuo. 0.1 N HCl was added to the mixture, causing a pale solid to form. The solid was filtered and dried. 1.1 grams of solid were obtained, resulting in an 88% yield.

Example III: 3-(3-carboxy-4-hydroxyphenyl)propanoic acid

0.52 grams 3-(3-carboxy-4-hydroxyphenyl)-propenoic acid were dissolved in 20 ml 1% sodium

hydroxide and reduced in the presence of 10% palladium on activated carbon at a hydrogen pressure of 2 psi for 4 hours. The catalyst was filtered and the filtrate was acidified with concentrated hydrochloric acid to pH 1. The solution was extracted with ether. The ether was removed in vacuo. The residue was crystallized from ether to yield 0.21g; mp 214-217°C.

Example IV:
4-(3-carboxy-4-hydroxyphenyl)-4-oxobutanoic acid

Salicylic acid (2g) and succinic anhydride (2g) were suspended in 80 ml carbon disulfide under a nitrogen atmosphere at room temperature. Aluminum chloride (8.8g) in 10ml of nitromethane was added dropwise. After the addition was completed, substantial hydrochloride gas evolved. After 5 minutes, the mixture was heated to reflux for 16 hours. After cooling to room temperature, 100 ml of water were added. The solvent was removed in vacuo. Water (50 ml) and 6 ml concentrated hydrochloric acid were added, causing a solid to form. The mixture was extracted with ether. The ether was dried and decolorized by heating with decolorizing charcoal. After filtering, the filtrate was reduced in vacuo. A yellow residue resulted (2.7g). The residue contained a small amount of salicylic acid that could be removed by crystallization from ether.

Example V: 4-(3-carboxy-4-hydroxyphenyl)butanoic acid

4-(3-carboxy-4-hydroxyphenyl)-4-oxobutanoic acid (0.5g) and sodium hydroxide (0.28g) were dissolved in 5 ml water. Sodium borohydride (1g) and 5 ml water were added. The reaction was refluxed for 43 hours. After cooling to room temperature, the reaction was acidified to pH 1 by dropwise addition of 6N hydrochloric acid and enough water to allow stirring to continue. The resulting precipitate was filtered. The filtrate was extracted with ether and the ether was removed in vacuo. The yield was 0.33 of product.

Example VI: 5-Formyl-2-hydroxybenzoic acid oxime

5-Formyl-2-hydroxybenzoic acid (1g, Aldrich) and hydroxylamine hydrochloride (0.5g) were dissolved in 100 ml 50% methanol/water. Sodium carbonate (0.5g) in 10 ml $H_2O$ was added dropwise over 5 minutes at room temperature and the reaction was stirred for 17 hours. The solvent was removed in vacuo. Water and ether were added and stirred until the residue had dissolved. The layers were separated and the water layer was extracted with ether. The ether was removed in vacuo to yield 0.92g of a pale solid.

Example VII: 5-(Aminomethyl)-2-hydroxybenzoic acid

5-Formyl-2-hydroxybenzoic acid oxime (0.5g) in 100 ml methanol was reduced in the presence of 20% palladium on activated carbon (0.10g) at room temperature and a pressure of 3 atom of hydrogen for 4 hours. The catalyst was removed by filtration and the filtrate was reduced in vacuo. The residue was suspended in a small amount of methanol and the pale gray solid was filtered and dried to yield 0.37g.

Example VIII: 6-[[{(3-Carboxy-4-hydroxyphenyl)methyl}-amino]carbonyl] fluorescein

6-Carboxyfluorescein (10mg, Calbiochem), N-hydroxysuccinimide (3mg) and N,N'-dicyclohexylcarbodiimide (8 mg) were dissolved in 0.3 ml dry pyridine with stirring at room temperature for 25 minutes. 5-(Aminomethyl)-2-hydroxybenzoic acid (7mg) was added and the mixture was stirred 3 days in a stoppered flask. The product was obtained by purification on silica gel preparative plates eluted with the appropriate mixture of chloroform, methanol and acetic acid. The correct band was repurified on silica gel preparative plates eluted with the appropriate mixture of ethyl acetate, methanol and acetic acid.

Example IX: 5-[[4-{(3-carboxy-4-hydroxyphenyl)methyl}-amino-6-chloro-1,3,5-triazin-2-yl]amino]fluorescein

5-(Aminomethyl)-2-hydroxybenzoic acid (17mg) and 5-((4,6-dichloro-1,3,5-triazin-2-yl)amino)fluorescein (50 mg) were dissolved in 2 ml methanol and 0.1 ml triethylamine and stirred at room temperature for 16 hours in a stoppered flask. The product was obtained by purification on silica gel preparative plates eluted with the appropriate mixture of chloroform, methanol and acetic acid. The correct band was repurified on silica gel preparative plates eluted with the appropriate mixture of ethyl acetate, methanol and acetic acid.

Example X: 5-[{(2-(3-carboxy-4-hydroxyphenyl)ethen-1-yl}carbonyl}amino]fluorescein

3-(3-Carboxy-4-hydroxyphenyl)propenoic acid (0.103g), N,N'-dicyclohexyl carbodiimide (0.11g) and 1-hydroxybenzotriazole (0.071g) were dissolved in 2 ml dry pyridine with stirring. After 20 minutes, 5-aminofluorescein (0.173g) was added and stirring in a stoppered flask was continued for 7 days. The product was obtained by purification on silica gel preparative plates eluted with the appropriate mixture of chloroform, methanol and acetic acid.

Example XI: 5-[{4-(3-carboxy-4-hydroxyphenyl)amino-6-chloro-1,3,5-triazin-2-yl}amino]fluorescein

5-Aminosalicylic acid (7.6 mg) and DTAF (isomer I) (26.6 mg) were dissolved in methanol. After

0194352

10 minutes, the reaction was complete. The product was purified on silica gel preparative plates eluted with the appropriate mixture of chloroform and methanol.

Examples XII through XXV describe how various precursors for immunogens and tracers can be produced in accordance with the principles of this invention.

Example XII: 2-Fluoro-5-methylbenzoic acid:

2-Amino-5-methylbenzoic acid (41 mmol) (Aldrich) is dissolved in 10 ml 60% fluoboric acid (Aldrich) and 50 ml water and cooled to -6°C. Sodium nitrate (44 mmol)(Aldrich) in 25 ml water is added to the stirred solution. Cuprous chloride (3 mmol) (Aldrich) is added and the reaction is heated to reflux for one hour. After cooling to room temperature, the solvent is removed *in vacuo*. The residue is dissolved in aqueous sodium carbonate and the product is precipitated by addition of concentrated hydrochloric acid. The solid is filtered and is crystallized from benzene.

Example XIII: Bromination of the substituted toluic acid

[Fig 10: Y=OH,$NO_2$,Cl or F; W=OH; $Z_1$,$Z_2$,$Z_3$=H or F; R=$CH_2$; X=Br]

The substituted toluic acid (0.27 mol) is dissolved in one liter of carbon tetrachloride and is heated to reflux. N-Bromosuccinimide (NBS) (0.28 mol) is added portionwise with stirring and irradiation with a 100-W tungsten lamp over three hours. After complete addition of NBS, the reaction is refluxed for three additional hours. After cooling to room temperature,

the succinimide that formed in the reaction is filtered and the filtrate is reduced in vacuo. The residue is crystallized from ether.

Example XIV: Oxidation of the α-bromotoluic acid derivatives

[Fig 10: Y=OH,$NO_2$,Cl or F; W=OH; $Z_1$,$Z_2$,$Z_3$=H or F; R=none; X=CHO]

Sodium metal (0.4 mol, for X=OH use 0.6 mol) is added to 200 ml methanol at 0°C and allowed to warm to room temperature. 2-nitropropane (0.21 mol) is added and the mixture is stirred for 30 minutes. The α-bromotoluic acid (0.19 mol) in methanol is added dropwise over 10 minutes and the reaction is stirred for an additional 2 hours. Remove the solvent in vacuo. Dissolve the residue in 5% sodium carbonate and acidify with concentrated hydrochloric acid. The precipitate is filtered and crystallized from ether or benzene.

Example XV: 5-(Hydroxymethyl)-2-methylbenzoic acid

The procedure that is applicable to this reaction is contained in M. Nakazaki et al. J. Org. Chem. 1980, 45, 1428-1435 with complete details.

Example XVI: 5-Formyl-2-methylbenzoic acid

5-Hydroxymethyl-2-methylbenzoic acid (0.1 mol) and manganese dioxide (0.4 mol) are suspended in 500 ml benzene. The mixture is heated to reflux with a water separator for about 16 hours. Filter the reaction through celite after cooling to room temperature. Remove the solvent in vacuo. The residue is crystallized from benzene.

0194352

Example XVII: (Methoxymethyl)triphenylphosphonium bromide

Triphenylphosphine (0.40 mol) and Bromomethoxymethane (0.40 mol) are dissolved in 200 ml dry benzene and heated to reflux with stirring for about 16 hours. After cooling to room temperature, stir for about 16 hours. Filter the solid that forms and wash the solid with benzene. Dry the solid *in vacuo*.

Example XVIII: 5-(2-Methoxyethenyl)benzoic acid derivatives

[Fig 10: Y=OH, $NO_2$, Cl, F or $CH_3$; W=OH; $Z_1$, $Z_2$, $Z_3$=H or F; R=CH=CH; X=$CH_3$]

The 5-formylbenzoic acid derivative (27 mmol) and methoxymethyltriphenylphosphonium bromide (39 mmol) are dissolved in 150 ml dry methanol under a nitrogen atmosphere. Lithium metal (80 mmol; for X=OH 120 mmol) in 70 ml dry methanol is added dropwise over 10 minutes. Monitor the reaction by silica gel TLC using a mixture of methanol and chloroform as eluting solvent. After about 3 days, the solvent is removed *in vacuo*. The residue is dissolved in water and acidified with concentrated hydrochloric acid. The water layer is exhaustively extracted with ether. The ether is dried and removed *in vacuo*. The product is purified by chromatography on silica gel using the appropriate methanol/chloroform mixture.

Example XIX: 5-(Formylmethyl)benzoic acid derivatives

[Fig 10: Y=OH, $NO_2$, Cl, F or $CH_3$; W=OH; $Z_1$, $Z_2$, $Z_3$=H or F; R=$CH_2$; X=CHO]

The 5-(2-methoxyethen-1-yl)benzoic acid derivative is dissolved in 250 ml 3N perchloric acid and 250 ml tetrahydrofuran. The mixture is stirred at room temperature for about 3 hours and then extracted with ether. The ether is dried and removed *in vacuo*. The residue is crystallized from ether or benzene.

Example XX: (1,3-dioxolan-2-ylmethyl)triphenylphosphonium bromide

2-(Bromomethyl)-1, 3-dioxolane and triphenylphosphine are coupled by the procedure outlined for methoxymethyl triphenyl phosphonium bromide (Example XVI).

Example XXI: 5-[-2-(1,3-dioxolan-2-yl)ethen-1-yl]benzoic acid derivatives

[Fig 10: Y=OH,$NO_2$,Cl,F or $CH_3$; W=OH; $Z_1$,$Z_2$,$Z_3$=H or F; R=CH=CH; X=1,3-dioxolan-2-yl]

(1,3-Dioxolan-zylmethyl)triphenylphosphonium bromide is coupled to the 5-formyl benzoic acid derivative by the procedure outlined for the 5-(2-methoxyethenyl)benzoic acid derivatives (Example XVIII).

Example XXII: 5-(3-oxopropen-1-yl)benzoic acid derivatives

[Fig 10: Y=OH,$NO_2$,Cl,F or $CH_3$; W=OH; $Z_1$,$Z_2$,$Z_3$=H or F; R=CH=CH; X=CHO]

The 5[(1,3-dioxolan-2-yl)ethen-1-yl]benzoic acid derivatives are hydrolyzed by the procedure outlined for the 5-(formylmethyl)benzoic acid derivatives (Example XIX).

Example XXIII: 5-(3-oxopropan-1-yl)benzoic acid derivatives

[Fig 10: Y=OH, $NO_2$, F, Cl or $CH_3$; W=OH; $Z_1$, $Z_2$, $Z_3$=H or F; R=$CH_2CH_2$; X=CHO]

The 5-(3-oxopropen-1-yl)benzoic acid derivative (0.2 mol) in 200 ml ethanol (for X=$NO_2$ add 1.5 mol equiv. of conc. HCl) is reduced over 1% by weight platinum oxide at room temperature and a pressure of 1.5 atm of hydrogen for about one hour. The catalyst is removed by filtration and the solvent is removed *in vacuo*. The residue is crystallized from alcohol.

Example XXIV: 5-(3-oxopropen-1-yl)benzoic acid oxime derivatives

[Fig 10: Y=OH, $NO_2$, F, Cl or $CH_3$; W=OH; $Z_1$, $Z_2$, $Z_3$=H or F; R=CH=CH; X=CH=NHOH]

The 5-(3-oxopropen-1-yl)benzoic acid derivative (0.2 mol) and hydroxylamine hydrochloride (0.24 mol) are dissolved in 250 ml water and 250 ml methanol. Sodium carbonate (0.15 mol) in 150 ml water is added dropwise over 20 minutes at room temperature with stirring. After the addition is complete, the reaction is stirred for about 17 hours. The volume is reduced *in vacuo* and the mixture is extracted with ether. The ether is washed with saturated aqueous sodium chloride, dried and removed *in vacuo*. The residue is crystallized from ether or benzene.

Example XXV: 5-(3-aminoprop-1-yl) benzoic acid derivatives

[Fig 10: Y=OH,$NH_2$,F,Cl or $CH_3$; W=OH; $Z_1$,$Z_2$,$Z_3$=H or F; R=$CH_2CH_2CH_2$; X=$NH_2$]

The 5-(3-oxopropen-1-yl)benzoic acid oxime derivative (0.2 mol) in 200 ml acetic acid is reduced over 1% by weight platinum oxide at room temperature and a pressure of 1.5 atm of hydrogen for about two hours. The catalyst is removed by filtration and the solvent is removed *in vacuo*. The residue is crystallized from alcohol.

CLAIMS

1. A compound comprising the structure:

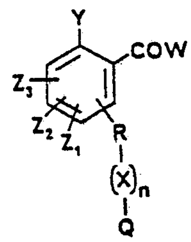

wherein

Q is a poly(amino acid), a poly(amino acid) derivative, fluorescein, or a fluorescein derivative;

X is NH or CO;

n is 0, 1 or 2;

R can be attached to any of the carbon atoms in the benzene ring other than those carbon atoms attached to Y or COW, and where R is a linking group including up to 2 heteroatoms when Q is a poly(amino acid) or a poly(amino acid) derivative and up to 4 heteroatoms having a total of from 0 to 8 carbon atoms and heteroatoms;

W is OH, or any OH salt;

Y is OH, $NH_2$, $CH_3$, F, Cl, Br or H when Q is fluorescein or a fluorescein derivative, and Y is OH, $NH_2$, $CH_3$, F or Cl when Q is a poly(amino acid) or a poly(amino acid) derivative; and

$Z_1$, $Z_2$ and $Z_3$ are each independently H or F when Q is fluorescein or a fluorescein derivative, and are each H when Q is a poly(amino acid) or a poly(amino acid) derivative.

2. The compound of Claim 1 wherein Q is bovine serum albumin.

3. The compound of Claim 1 wherein Q is an amino, an amido, an amidino, a urea, a thiourea, a carbamate, a thiocarbamate, a triazinylamino, or a (carboxyamino)-sulfonamido derivative of fluorescein.

4. An antibody to a compound according to Claim 1 when Q is a poly(amino acid) or a poly(amino acid) derivative.

5. A method for making an immunogen comprising the step of coupling a precursor of the formula

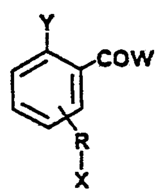

where:

R can be attached to any of the carbon atoms in the benzene ring other than those carbon atoms attached to Y or COW, and where R is a linking group including up to 2 heteroatoms and having a total of from 0 to 8 carbon atoms and heteroatoms;

W is OH, or any OH salt;

X is $NH_2$, COOH, CN, CHO, Br, I or OH; and

Y is OH, $NH_2$, $CH_3$, F or Cl;

with a poly(amino acid) or a derivative of a poly(amino acid).

6. The method of Claim 5 wherein when X is $NH_2$ or $CO_2H$, the precursor is coupled by reacting it with the poly(amino acid) by addition of N, N'-dicyclohexyl-carbodiimide, 1-ethyl-3-(3-dimethyl-aminopropyl)carbodiimide or 1-cyclohexyl-3-(2-morpholinoethyl)-carbodiimide metho-p-toluenesulfonate.

7. A method for making a tracer comprising the step of coupling a precursor of the formula

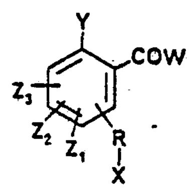

where:

R can be attached to any of the carbon atoms in the benzene ring other than those carbon atoms attached to Y or COW, and where R is a linking group including up to 4 heteroatoms and having a total of from 0 to 8 carbon atoms and heteroatoms;

W is OH, or any OH salt;

X is $NH_2$, COOH, CN or OH;

Y is OH, $NH_2$, $CH_3$, F, Cl, Br or H;

and

$Z_1$, $Z_2$ and $Z_3$ are each independently H or F; with fluorescein or a derivative of fluorescein.

8. A process for measuring concentration of salicylate which comprises the steps of:

(a) contacting a sample with a salicylate antiserum and with a compound according to Claim 1 capable of producing a detectable fluorescence polarization response to the presence of the salicylate antiserum;

(b) passing plane polarized light through the resulting solution from step (a) to obtain a fluorescence polarization response; and

(c) detecting the fluorescence polarization response of the solution of step (b) as a measure of the amount of salicylate in the sample.

U. S. PATENT AGENTS - EUROPEAN PATENT ATTORNEYS

D-8000 MÜNCHEN 5 (WEST GERMANY)
BAADERSTR. 3 - PHONE (89) 221.328

I-20123 MILANO (ITALY)
VIA MERAVIGLI, 16 - P...

PHONE FROM USA (01202) 670.360 OR (01339) 22,12,18
TELEX: 896.840 PATMOI FROM USA: 048.884.840

DR. ING. G. MODIANO
DR. ING. A. JOSIF
DR. ING. M. PISANTY*
DR. ING. O. STAUB*
DR. ING. H. DE CARLI*
DR. ING. N. ZANOTTI*
C. S. RENIERO*
S. L. A. MODIANO*

*EUROP. PATENT ATTORNEYS

A DIVISION OF
MODIANO & ASSOCIATI
VIA MERAVIGLI, 16 - MILAN

0194352

JAN 7 1986

EPA-EPO-OEB
MÜNCHEN
Empfang bestätigt
Receipt acknowledged
Accusé reception
HH

EPA EPO-OEB
DG 1
Reçu:
23 JAN. 1986

EUROPÄISCHES PATENTAMT
Erhardtstr 27
D-8000 MÜNCHEN 2
GERMANIA OCC.LE

For the purpose of publication, correction(s) ☒ allowed
☐ allowed with exception of the deleted points
☐ not allowed
Signature: date: 4/7/86
Receiving Section

Re: European patent application N. 85116063.0
filed on Dec. 17, 1985
ABBOTT LABORATORIES
Our ref. 48085/GM/ms

Dear Sirs,

Applicant herewith files five sheets of drawings which it has erroneously omitted to add to the papers of the above referred application, as filed on 17 December 1985.

Applicant respectfully asks that this belated filing of such drawings be considered as made within the frame of a "request (of correction) under Rule 88 of the EPC" and in support of this request, submits the following:

- According to Rule 88, "mistakes in any document filed with the EPO may be corrected on request ...; if the request for such correction concerns ... drawings, the correction must be evident in the sense that it is immediately evident that nothing else would have been intended than what is offered as the correction".

- According to the landmark Decision of 18 July 1980 J 08/80 of the Legal Board of Appeal, published in the Official Journal EPO, at page 293 and the following ones and more precisely according to the 4th "Reason for the Decision" "The mistake ... may result from an omission. Correction, accordingly, can take the form of ... adding omitted matter."

- According to the same landmark decision "5. Before the Office can accede to a request for correction of a mistake, however, it must be satisfied that a mistake was made, what

the mistake was and what the correction should be. This is the necessary safeguard against abuse of the provisions of Rule 88 EPC".

- Again according to said Decision "6. ... there should be no reasonable doubt as to the true intention of the person on whose behalf the document was filed".

- Even the decision of 7 April 1982-J 01/82- of the Legal Board of Appeal, dealing with late filing of drawings, based the rejection of the correction essentially on the fact that "the danger (of an addition of new subject matter-added reference) exists if one or more figures are missing", a danger which in the instant case does not come into being, as will be demonstrated.

- In connection with such a danger, it is obvious that EPO has to nourish doubts when it finds out, as has been the case with the circumstances depicted in the Decision of the Legal Board of Appeal dated 18 Oct. 1982, J 13/82, published in the Official Journal EPO 1/1983, pages 12 and the following ones, that "contrary to the appellant's assertion, (an omitted document) was not included in the certified copy of the US priority document."
Again such doubts do not exist in the instant case, as will be demonstrated.

- Now, with this law situation as a background, Applicant submits that:

(a) the circumstance that no hidden intention existed to add new matter and that just an error (omission of filing) occurred is clearly demonstrated by the fact that on pages 6 and 7 of the specification as filed, a direct reference was already made - at the time of filing - to the "following figures"- which are also shown to comprise "Fig. 1 to 20". This clearly shows that the failure of filing is due to an obvious error and by no means to a different intention of the Applicant. The error is inter alia due to the fact that the drawings referred to are not, at a first glance, corresponding to the conventional notion of patent drawings, inasmuch as each figure relates to a chemical formula, a fact which has generated an easily understandable confusion.

Accordingly, the first condition set forth in said landmark Decision J 08/80, according to which the EPO should be satisfied that a mistake was made, is fully met.

(b) also the second and third conditions, which require that the EPO should be fully knowledgeable about "what the mistake was " and "what the correction should be" are also fully met, since they reside "in re ipsa", in the omission to file the drawings, and in the desire to complete the specification, by a systematic and graphical, more easily accessible representation of the compounds which the specification already considers essentially with explicit language, by the use of the chemical names.

(c) the fact that no danger exists that any new matter could or would be added by adding the drawings to the specification is proven by the following two circumstances:

(i) the contents of each of the Figures making up the drawings are already included in the specification as filed with the EPO on the 17th December 1985, with sufficient language to identify each figure itself, to such an extent that in substance, such drawings only help in having a clearer systematical accession to all the notions relied on in the specification, without adding anything to it. In other words, the specification is in substance self-supported on its own even without the help of the drawings, but the addition of the drawings makes more immediate its intelligence. Thus:

Fig. 1 is said, in the specification, page 6, line 19, to "show the general structure of the (known) class of salicylates", and Fig. 1 in the drawings just shows the conventional widely known structural formula of such class of salicylates.

Fig. 2 in the drawings is relating to a formula of reactants which is clearly reproduced in Claim 5.

Fig. 3 in the drawings is relating to a formula of a class of reactants which is clearly reproduced in claim 7.

Fig. 4 is said (in the specification, at page 6,line 28) to show "the alternate structural formulae and names of the fluorescein moiety", and the same formula as illustrated in the drawings appears to define fluorescein for inst. in the "Encyclopedia of Chemical Technology", by Kirk-Othmer, at page 769 (1967 Edition, volume 5) see enclosure A.

Fig. 5 in the drawing is relating to a formula of the

inventive compound which formula is clearly reproduced in claim 1.

Fig. 6 in the drawing is relating to the formula of the immunogens as defined in claim 1, where Q is a poly(amino)acid (which is one of the possible conditions of claim 1), see also page 4, lines 31 and 32 of the specification.

Fig. 7 in the drawing is the formula of the tracers referred to at page 11, lines 23 to 26.

Fig. 8 in the drawings is the formula of a particular embodiment of the immunogen compound defined in Fig. 6, an embodiment which is referred to in the specification, on page 11, lines 1 to 4.

Fig. 9 in the drawings is a particular embodiment of the preferred tracer encompassed in the formula of Fig. 7, an embodiment which is illustrated in the specification on page 11, lines 23 to 27.

Fig. 10 in the drawing is a precursor of the compound of Fig. 5 and Fig. 7 (claim 1), disclosed at the bottom of page 12 and the beginning of page 13.

Fig. 11 in the drawings illustrates linking groups between the tracers and the fluorescein derivatives, as explained on page 11, lines 28 to 32.

Fig. 12 in the drawings illustrates in graphical form the tracer specifically referred to in Example VIII.

Fig. 13 in the drawings illustrates in graphical form an isomer of the compound shown in Fig. 12.

Fig. 14 in the drawings illustrates in graphical form the tracer referred to in Example IX.

Fig. 15 in the drawing illustrates in graphical form the tracer referred to in the Example X.

Fig. 16 in the drawing illustrates in graphical form the tracer referred to in Example XI.

Fig. 17 in the drawing illustrates in graphical form the precursor referred to in example III.

Fig. 18 in the drawings illustrates in graphical form the precursor referred to in example V.

Fig. 19 in the drawing illustrates in graphical form the starting compound mentioned in Example XI.

Fig. 20 in the drawing illustrates in graphical form the precursor referred to in Example II.

(ii) the second circumstance resides in the fact that in the Certified Copy of the US specification (which has been filed in support of the priority-claim generating U.S. application dated December 19, 1984) all such drawings with all such figures are already present, a fact that easily shows that Applicant had no intention to add new matter, invented at a later time, to the specification as filed with EPO.

(d) the above facts show that "the correction is obvious in the sense that it is immediately evident that nothing else would have been intended than what is offered as the correction", a language excerpted from Rule 88, but which so clearly applies to the instant case as to constitute a paradigmatic illustration of the language itself.

In view of the above arguments, Applicant believes to be entitled to the correction of the erroneous omission, and a favourable communication in this respect is respectfully solicited.

Respectfully submitted,

for ABBOTT LABORATORIES

Dr. G. Modiano

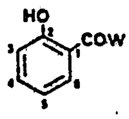

FIG. 1

W=OH or OH Salts

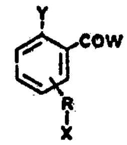

FIG. 2

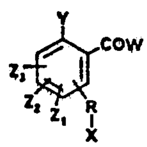

FIG. 3

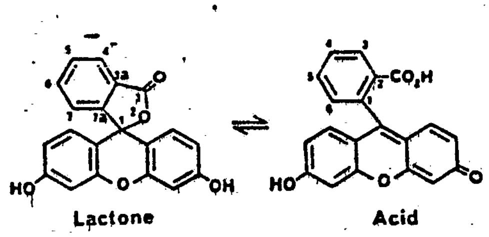

Lactone

Acid

FIG. 4

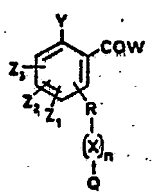

FIG. 5

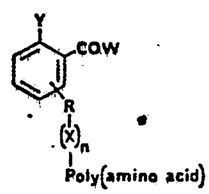

FIG. 6

For the purpose of publication, correction(s)
☒ allowed
☐ allowed with exception of the deleted points
☐ not allowed
Signature: date: 3/7/86
Receiving Section

2/5

0194352

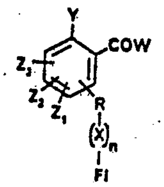

FIG. 7

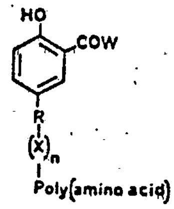

FIG. 8

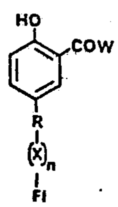

FIG. 9

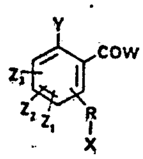

FIG. 10

For the purpose of publication, correction(s)
☒ allowed
☐ allowed with exception of the deleted points
☐ not allowed
Signature: date: 3-7-86
Receiving Section

FIG. 11

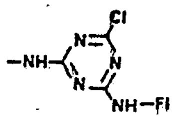

FIG. 11-1

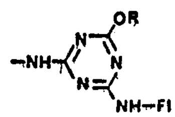

FIG. 11-2

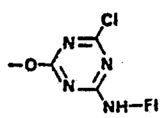

FIG. 11-3

-NH-CO-Fl

FIG. 11-4

-CO-NH-Fl

FIG. 11-5

-CNH-NH-Fl

FIG. 11-6

-NH-CO-NH-Fl

FIG. 11-7

-NH-CS-NH-Fl

FIG. 11-8

-O-CO-NH-Fl

FIG. 11-9

-O-CS-NH-Fl

FIG. 11-10

For the purpose of publication, correction(s)
☒ allowed
☐ allowed with exception of the deleted points
☐ not allowed
Signature: date: 3-7-06
Receiving Section

0194352

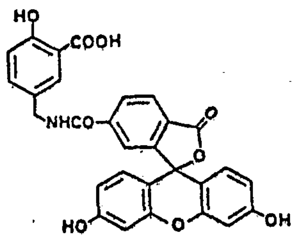

FIG. 12

6-[[{(3-Carboxy-4-hydroxyphenyl)methyl} amino] carbonyl] fluorescein

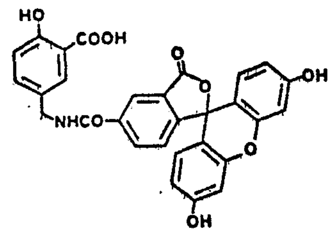

FIG. 13

5-[[{(3-Carboxy-4-hydroxyphenyl)methyl} amino] carbonyl]-fluorescein

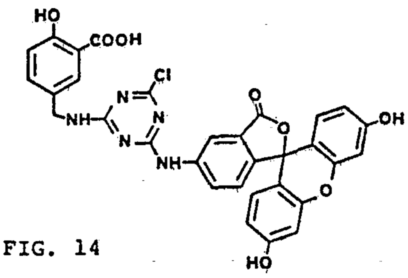

FIG. 14

5-[[4-{(3-Carboxy-4-hydroxyphenyl)methyl} amino-6-chloro-1,3,5-triazin-2-yl]-amino] fluorescein

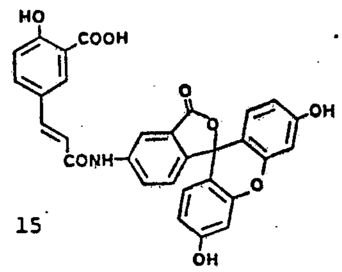

FIG. 15

5-[{(2-(3-Carboxy-4-hydroxyphenyl) ethen-1-yl)carbonyl} amino] fluorescein

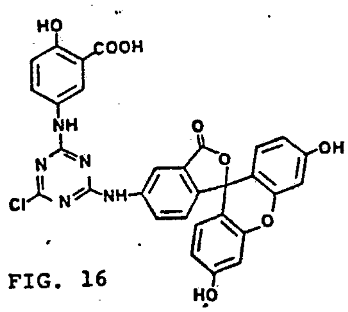

FIG. 16

5-[{4-(3-Carboxy-4-hydroxyphenyl)amino-6-chloro-1,3,5-triazin-2-yl} amino]-fluorescein

For the purpose of publication, correction(s)
☒ allowed
☐ allowed with exception of the deleted points
☐ not allowed
Signature: date: 3/7/86
Receiving Section

0194352

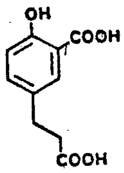

FIG. 17

3-(3-Carboxy-4-hydroxyphenyl)-propanoic acid

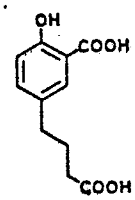

FIG. 18

4-(3-Carboxy-4-hydroxy-phenyl)butanoic acid

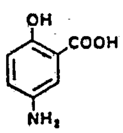

FIG. 19

5-Aminosalicylic acid

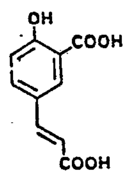

FIG. 20

3-(3-Carboxy-4-hydroxyphenyl)-propenoic acid

For the purpose of publication, correction(s)

☒ allowed

☐ allowed with exception of the deleted points

☐ not allowed

Signature: date: 3/7/86

Receiving Section